Europäisches Patentamt

European Patent Office

Office européen des brevets

(11) Publication number: **0 484 767 A1**

## EUROPEAN PATENT APPLICATION

(21) Application number: **91118233.5**

(22) Date of filing: **25.10.91**

(51) Int. Cl.⁵: **C07C 209/36**

(30) Priority: **05.11.90 US 608912**

(43) Date of publication of application:
**13.05.92 Bulletin 92/20**

(84) Designated Contracting States:
**AT BE CH DE FR GB IT LI LU NL**

(71) Applicant: **OCCIDENTAL CHEMICAL CORPORATION**
**2801 Long Road**
**Grand Island New York 14072-1244(US)**

(72) Inventor: **Krishnamurti, Ramesh, Occidental Chemical Corp.**
**Patent Department, 2801 Long Road**
**Grand Island, New York 14072(US)**
Inventor: **Fertel, Lawrence B., Occidental Chemical Corp.**
**Patent Department, 2801 Long Road**
**Grand Island, New York 14072(US)**
Inventor: **Lin, Henry C., Occidental Chemical Corp.**
**Patent Department, 2801 Long Road**
**Grand Island, New York 14072(US)**
Inventor: **Dosi, Mahendra K., Occidental Chemical Corp.**
**Patent Department, 2801 Long Road**
**Grand Island, New York 14072(US)**

(74) Representative: **Vossius & Partner**
**Siebertstrasse 4 P.O. Box 86 07 67**
**W-8000 München 86(DE)**

(54) **Preparation of 3,5-diamino benzotrifluoride.**

(57) 3,5-Diaminobenzotrifluoride may be produced, in a single step, by reacting 4-chloro-3,5-dinitrobenzotrifluoride, in a suitable solvent, with hydrogen gas, in the presence of a catalyst comprising palladium on a suitable carrier.

EP 0 484 767 A1

This invention relates to a process for the preparation of 3,5-diamino benzotrifluoride by the reduction of 4-chloro-3,5-dinitro benzotrifluoride using hydrogen as a reducing agent in the presence of palladium on charcoal as a catalyst.

The reduction of aromatic nitro compounds containing halogen on the aromatic ring is unpredictable. Hydrogenation using a palladium on charcoal catalyst generally reduces the nitro group to an amine. However, the hydrogenation has been reported to fail in some cases. In addition, the effects of such hydrogenation upon a ring halogen are unpredictable. Occasionally the ring halogen is removed from the ring and replaced by a hydrogen. However, in many cases the hydrogenation leaves the ring halogen intact.

A. Weizmann discloses in J. Am. Chem. Soc., 71, 4154 (1949), that the catalytic hydrogenation of diethylaminoethyl 4-nitro-2-chlorobenzoate using palladium on barium sulfate as a catalyst was impractical from a preparative point of view. The nitro compound was often incompletely reduced. The chlorine was occasionally removed from the ring while in other reactions it remained on the ring. Similar hydrogenation experiments conducted with 4-nitro-2-chlorobenzoic acid and its ethyl ester, using palladium on barium sulfate as a catalyst, produced variable results depending upon the solvent employed. In ethyl acetate, the reduction proceeded with retention of the chlorine and the acid and the ethyl ester gave quantitative yields of 4-amino-2-chlorobenzoic acid and ethyl 4-amino-2-chlorobenzoate respectively. In isopropyl alcohol, the 4-nitro-2-chlorobenzoic acid and its ethyl ester were reduced to 4-aminobenzoic acid and its ethyl ester respectively. In other words, the chlorine was lost in the reduction process. An aqueous solution of sodium 4-nitro-2-chlorobenzoate yielded, on work-up, 4-aminobenzoic acid.

Bouchet et al disclose in Syn. Common., 4, 57-9 (1974) that paranitrochlorobenzene may be reduced to parachloroaniline using hydrogen in the presence of a palladium on carbon catalyst.

Ovchinnikov et al (in Prikl. Khim., (62), 37-44 (1969)) that meta- and para-chloronitrobenzenes may be hydrogenated to meta- and para-chloroanilines respectively using hydrogen gas and a 2% palladium on carbon catalyst. Approximately 2% dehalogenation was observed. It was also observed that the amount of dehalogenation was related to the type of carbon used as the catalyst carrier and depended upon the height of the catalyst bed.

U.S. Patent 3,666,813 discloses that aromatic haloamines may be prepared by hydrogenating the corresponding chloronitro aromatic compound in the presence of a modified palladium on charcoal catalyst. The palladium on carbon catalyst is modified by treating it with a solution of a bismuth, lead or silver salt.

European Patent Application EP 88667 (as abstracted in Chem. Abstracts 100:52475m and Derwent accession #C83-089930) discloses that chlorinated or brominated methylenedianilines may be prepared by the nitration and reduction of the corresponding aromatic halides. The reduction is carried out in methanol solvent with hydrogen gas in the presence of 5% palladium on carbon as a catalyst. The aromatic halide is retained during reduction.

Chakrabarti et al disclose in two papers (J. Med. Chem. 23 p. 878 and 884 (1980)) a multi-step reduction reaction in which the first step is a hydrogenation using 10% palladium on carbon as a catalyst. The molecules which are subjected to hydrogenation are substituted nitrobenzenes with a halogen at the 3-position and a substituted amino group at the 6-position. In one paper the compound studied has fluorine as the halogen and in the other the halogen is chlorine. In each case, the nitro group was reduced to the amine while the halogen was not attacked.

Japanese Patent 63/010739 (as abstracted in Chem. Abstracts 109:92449y) dislcoses that chlorofluoro-benzotrifluoride derivatives may be dechlorinated using hydrogen gas and 5% palladium charcoal catalysts in a methanol solvent. The ring chlorines are preferentially removed over the ring fluorines.

Vergnani et al disclosed in Helv. Chim. Acta. 68, 1828, (1985), that 5-bromo-2-methyl-8-nitro-1,2,3,4-tetrahydroisoquinoline undergoes simultaneous removal of the aromatic bromine and reduction of the nitro group to an amine when treated with hydrogen gas in the presence of a 10% palladium on charocal catalyst, triethyl amine and methanol as a solvent.

Japanese Patent 58157749 (as abstracted in Chem. Abstracts 100:51247b) discloses that 2,2'4-trichloro-4',5-dinitrodiphenyl ether may be hydrogenated in the presence of 5% palladium on carbon catalyst in methanol to form 3,4'-diaminodiphenyl ether. The substance which is reduced using the process of this invention, that is 4-chloro-3,5-dinitro benzotrifluoride, is a rather reactive molecule and can undergo side reactions during any reduction process.

Crampton and Greenhalgh have disclosed, in J. Chem. Soc., Perkins Transaction II, p. 187 (1986), that 4-chloro-3,5-dinitro benzotrifluoride is subject to nucleophilic attack on the chlorine. Thus, hydroxide ion can displace the chlorine to yield 4-hydroxy-3,5-dinitro benzotrifluoride.

Attempts were made in our laboratories to reduce 4-chloro-3,5-dinitro benzotrifluoride using sodium formate and a palladium on carbon catalyst. A variety of dipolar aprotic solvents and methanol were used. In all cases the reduction was either incomplete or did not occur at all and the products formed indicated that the 4-chloro group had been displaced to form either 4-hydroxy compounds, or in one case, a 4-methoxy compound. (See Comparative Examples 1-4.)

Surprisingly, it has been found that 3,5-Diaminobenzotrifluoride may be produced, in a single step, by reacting 4-chloro-3,5-dinitrobenzotrifluoride, in a suitable solvent, with hydrogen gas, in the presence of a catalyst comprising palladium on a suitable carrier. Optionally, a suitable base may be used in the reaction mixture. 3,5-Diaminobenzotrifluoride is a valuable intermediate used in the synthesis of polyimide polymers. The present process is advantageous in that the starting material, 4-chloro-3,5-dinitrobenzotrifluoride is commercially available.

3,5-Diaminobenzotrifluoride may be produced, in a single step, by reacting 4-chloro-3,5-dinitrobenzotrifluoride, in a suitable solvent, with hydrogen gas, in the presence of a catalyst comprising palladium on a suitable carrier. Optionally, a suitable base may be used in the reaction mixture. The reaction is conducted at moderate pressure; that is, about 50 to 300 psig. At the lower end, the pressure is not critical. Although the reaction can be run at pressures less than 50 psig, such reactions tend to be slow. Accordingly, it is preferred to run the reaction at about 50 psig or above. A hydrogen pressure of 300 psig is not an upper limit on the useable pressure, but is rather the upper end of the preferred region of operation.

The reaction may be conducted at moderate temperatures between room temperature and about 100°C. If the reaction is run at a temperature much lower than room temperature, it tends to be too slow to be useful. On the other hand, at temperatures about 100°C high molecular weight side products are formed.

Selection of the solvent is important in conducting this reaction. Nucleophilic solvents are not suitable for this process since they can attack the halogen and lead to unwanted side products. A particular example of this is methanol which can displace the chlorine and lead to the 4-methoxy compound as a product. This problem is illustrated in Comparative Example 4.

Dipolar aprotic solvents, such as dimethyl formamide, N-methyl-2-pyrrolidone, and dimethyl sulfoxide, are not suitable solvents because their use tends to lead to the formation of the hydroxy derivative. This is illustrated in Comparative Examples 1-3.

C-3 to C-6 secondary and tertiary alcohols, which are larger molecules than methanol, are suitable solvents because they cannot readily attack the somewhat stearically hindered 4-chloro group. Other suitable solvents include acetonitrile, ethers such as diethyl ether, tetrahydrofuran, ethylene glycol dimethyl ether (glyme), ethylene glycol dimethyl ether (glyme), triethylene glycol dimethyl ether (triglyme), and tetraethylene glycol dimethyl ether (tetraglyme), and low molecular weight esters of C-2 to C-4 alcohols with C-2 to C-4 acids such as, ethyl acetate, isopropyl acetate, propyl acetate, butyl acetates, ethyl proprionate, and ethyl butyrate. The most suitable solvent is ethyl acetate.

The use of a base in the reaction mixture is optional. However, the reaction tends to be rather slow in the absence of a base. The slowness is illustrated in Comparative Example 5, and Examples 5 and 6. Although we do not wish to be bound by theory, it is possible that a base speeds up the reaction by reacting with the hydrogen chloride formed when the chlorine is removed from the ring by attack of the hydrogen. In the absence of a base, the hydrogen chloride produced tends to poison the palladium catalyst. This diminishes the activity of the catalyst, and leads to incomplete conversion of starting material to product. It is well known that the chlorine group in the starting material is reactive toward nucleophiles. Accordingly, bases which are nucleophilic, such as the hydroxide ion, are to be avoided since such bases can readily attack the labile 4-chloro group and lead to the formation of undesirable products. Suitable bases include inorganic bases which are relatively insoluble in the chosen solvent, such as alkali and alkaline earth carbonates, and bicarbonates. In addition, the alkali metal salts of low molecular weight carboxylic acids are particularly useful as bases. The preferred base is sodium acetate.

The palladium catalyst may be used as a coating on any inert carrier. Barium sulfate is a suitable carrier. The preferred form for the catalyst is 5 to 10% coating on carbon.

The 3,5-diamino benzotrifluoride may be readily isolated and purified by methods such as solvent evaporation and recrystallization and other methods well-known to those skilled in the art.

EXAMPLES

Example 1

A solution of 4-chloro-3,5-dinitrobenzotrifluoride (DTI) (5.41 g, 0.02 mol) in ethyl acetate (40 mL) was charged in a Parr hydrogenator bottle and mixed with sodium acetate (1.64 g, 0.02 mol), and 5% Pd/C (0.5 g). After purging with nitrogen, hydrogen was charged periodically in the reaction bottle to maintain its pressure at 50 psig. The temperature inside the reactor was maintained at 40°C for 4 hours. After this duration, analysis of the reaction mixture by gas chromatography showed formation of 62.4% 3,5-diaminobenzotrifluoride (DABTF) and 34.9% 4-chloro-3,5-diaminobenzotrifluoride (CDABTF).

## Example 2

A solution of 4-chloro-3,5-dinitrobenzotrifluoride (DTI) (5.41 g, 0.02 mol) in ethyl acetate (40 mL) was charged in a Parr hydrogenator bottle and mixed with sodium acetate (1.64 g, 0.02 mol), and 5% Pd/C (0.505 g). After purging with nitrogen, hydrogen was charged periodically in the reaction bottle to maintain its pressure at 50 psig. The temperature inside the reactor was maintained at 100°C for 1.2 hours. After this duration, analysis of the reaction mixture by gas chromatography showed formation of 86.2% 3,5-diaminobenzotrifluoride (DABTF).

## Example 3

A solution of 4-chloro-3,5-dinitrobenzotrifluoride (DTI) (5.41 g, 0.02 mol) in ethyl acetate (40 mL) was charged in a Parr hydrogenator bottle and mixed with sodium acetate (2.46 g, 0.03 mol), and 5% Pd/C (0.5 g). After purging with nitrogen, hydrogen was charged periodically in the reaction bottle to maintain its pressure at 50 psig. The temperature inside the reactor was maintained at 100°C for 1 hours. After this duration, analysis of the reaction mixture by gas chromatography showed formation of 94.9% 3,5-diaminobenzotrifluoride (DABTF) and 1.3% 4-chloro-3,5-diaminobenzotrifluoride (CDABTF).

## Example 4

The reaction of Example 3 was repeated except that anhydrous sodium carbonate (3.18 g, 0.03 mol) was used instead of sodium acetate. After heating the reaction mixture for 1 hour at 100°C analysis of the reaction mixture by gas chromatography showed formation of 89.8% 3,5-diaminobenzotrifluoride (DABTF) and 7.2% 4-chloro-3,5-diaminobenzotrifluoride (CDABTF).

## Example 5

A solution of 4-chloro-3,5-dinitrobenzotrifluoride (15.0 g) in isopropanol (150 mL) was charged in a Parr hydrogenator bottle and mixed with 1.5 g of carbon supported palladium catalyst (5% Pd on carbon). After purging with nitrogen, hydrogen was charged periodically in the reaction bottle to maintain its pressure at 50 psig. The reaction temperature in the hydrogenator was kept at 45-100°C for 1 hour and then at 100°C for 2.5 hours. After this duration, the analysis of the reaction mixture by gas chromatography showed formation of 38.5% 3,5-diaminobenzotrifluoride (DABTF) and 59.5% 4-chloro-3,5-diaminobenzotrifluoride (CDABTF).

75 mL of the reaction mixture from end of the run was filtered to remove the catalyst. This was charged in the Parr hydrogenator reaction bottle and mixed with 0.75 g of fresh Pd/C catalyst. The reduction reaction with hydrogen was carried out at 100°C and 50 psig and after 4 hours the product analysis by gas chromatography showed 65.5% DABTF and 29.8% CDABTF.

## Example 6

A solution of 4-chloro-3,5-dinitrobenzotrifluoride (15.0 g) in isopropanol (150 mL) was charged in a 300 mL size autoclave and mixed with 1.5 g of carbon supported palladium catalyst (5% Pd on carbon). After purging with nitrogen, hydrogen was charged periodically into the autoclave to maintain its pressure at 100 psig while it was heating to 150°C during the initial 1/2 hour. The pressure was then increased to 150 psig with a continuous supply of hydrogen from a cylinder and the reaction temperature was maintained at 150°C by a temperature controller. After 6 hours, the analysis of the reaction mixture by gas chromatography showed formation of 55.3% 3,5-diaminobenzotrifluoride (DABTF), 6.3% 4-chloro-3,5-diaminobenzotrifluoride (CDABTF) and 27.1% of a ligher component which could be associated with the degradation of the solvent.

## COMPARATIVE EXAMPLES

## Comparative Example 1

A mixture of 4-chloro-3,5-dinitrobenzotrifluoride (1.0 g, 0.0037 mol), sodium formate (2.1 g, 0.0309 mol) and 10% palladium on carbon (Pd/C) (0.1 g) were stirred in 10 mL of dimethyl formamide (DMF) and heated to 50°C. After 1 hour, analysis by gas chromatography mass spectroscopy (GCMS) showed the formation of phenolic types of material, such as 3,5-dinitro-4-hydroxy benzotrifluoride and 3-amino-4-hydroxy-5-nitro benzotrifluoride. No 3,5-diamino benzotrifluoride (DABTF) was detected.

## Comparative Example 2

A mixture of 4-chloro-3,5-dinitrobenzotrifluoride (1.0 g, 0.0037 mol), sodium formate (2.49 g, 0.0309 mol) and 10% Pd/C (0.1 g) were stirred in 10 mL of NMP and heated to 115°C. After 1 hour, analysis by $^{19}$F NMR indicated 3-amino-4-hydroxy-5-nitro benzotrifluoride as the only fluorinated material formed.

Comparative Example 3

A mixture of 4-chloro-3,5-dinitrobenzotrifluoride (1.0 g, 0.0037 mol), sodium formate (2.53 g) and 10% Pd/C (0.1 g) were stirred in 10 mL of DMSO and heated to 115°C. After 1 hour, analysis by $^{19}$F NMR indicated 3-amino-4-hydroxy-5-nitro benzotrifluoride as the only fluorinated material.

Comparative Example 4

A mixture of 4-chloro-3,5-dinitrobenzotrifluoride (1.0 g, 0.0037 mol), sodium formate (2.08 g, 0.0306 mol) and 10% Pd/C (0.1 g) were stirred in 10 mL of methanol and heated to 70°C. After 1 hour, analysis by GCMS indicated 3,5-dinitro-4-methoxy benzotrifluoride as the only fluorinated material.

Comparative Example 5

A solution of 4-chloro-3,5-dinitrobenzotrifluoride (5.0 g) in isopropanol (50 mL) was charged in a Parr hydrogenator bottle and mixed with 0.5 g of carbon supported palladium catalyst (5% Pd on carbon). After purging with nitrogen, hydrogen was charged periodically in the reaction bottle to maintain its pressure at 50 psig. The reaction temperature in the hydrogenator was kept at 50°C for 4.5 hours and then at 75°C for 2 hours. After this duration, the analysis of the reaction mixture by gas chromatography showed formation of 23.7% 3,5-diaminobenzotrifluoride (DABTF) and 73.3% 4-chloro-3,5-diaminobenzotrifluoride (CDABTF).

Comparative Example 6

A solution of 4-chloro-3,5-dinitrobenzotrifluoride (5.0 g) in isopropanol (50 mL) was charged in a Parr hydrogenator bottle and mixed with 0.5 g of carbon supported palladium catalyst (5% Pd on carbon) and 1.1 g of sodium carbonate. After purging with nitrogen, hydrogen was charged periodically in the reaction bottle to maintain its pressure at 50 psig. The reaction temperature in the hydrogenator was kept at 50°C for 5 hours and then at 75°C for 2 hours. After this duration, the analysis of the reaction mixture by gas chromatography showed formation of 29.1% 3,5-diaminobenzotrifluoride (DABTF) and 68.1% 4-chloro-3,5-diaminobenzotrifluoride (CDABTF).

**Claims**

1. A process for the preparation of 3,5-diaminobenzotrifluoride which comprises treating 4-chloro-3,5-dinitrobenzotrifluoride, in a suitable solvent, with hydrogen gas in the presence of a catalyst which comprises palladium on a suitable carrier.

2. A process according to claim 1 in which the solvent is selected from the group consisting of C-3 to C-6 secondary and tertiary alcohols.

3. A process according to claim 1 or 2 in which the solvent is isopropyl alcohol.

4. A process according to claim 1 in which the solvent is an ether.

5. A process according to claim 1 in which the solvent is a low molecular weight ester.

6. A process according to claim 5 in which the solvent is ethyl acetate.

7. A process according to any one of claims 1 to 6 in which the catalyst is palladium on carbon.

8. A process according to any one of claims 1 to 7 conducted in the presence of a base.

9. A process according to claim 8 in which the base is sodium carbonate.

10. A process according to claim 9 in which the base is sodium acetate.

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 038 465 (HOECHST AG.) <br> * page 6, line 1 - page 7, line 30 * <br> * claims; examples * <br> --- | 1-10 | C07C209/36 |
| Y | JOURNAL OF THE CHEMICAL SOCIETY. <br> 1949, LETCHWORTH GB <br> pages 3016 - 3020; <br> W.B. WHALLEY: 'Organic Fluoro-compounds. Part I. Hydroxy-derivatives of Benzotrifluoride' <br> * page 3018, paragraph 6 * <br> ----- | 1-10 | |
| | | | **TECHNICAL FIELDS SEARCHED (Int. Cl.5)** <br><br> C07C |

The present search report has been drawn up for all claims

| Place of search | Date of completion of the search | Examiner |
|---|---|---|
| THE HAGUE | 11 FEBRUARY 1992 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)